# EUROPÄISCHE PATENTANMELDUNG

(11) **EP 3 130 316 A1**
(43) Veröffentlichungstag der Anmeldung: **15.02.2017**
(21) Anmeldenummer: 16179664.4
(22) Anmeldetag: 15.07.2016
(51) Int. Cl.: A61F 2/24, A61F 2/966, A61F 2/97

(54) **EINFÜHRKATHETER, KATHETERHÜLLE UND KATHETERANORDNUNG**

(30) Priorität: 13.08.2015 DE 102015010515
(71) Anmelder: Biotronik AG, 8180 Bülach (CH)
(72) Erfinder: Rothstock, Stephan, 18057 Rostock (DE); Koop, Karsten, 18057 Rostock (DE); Goebel, Paul, 18059 Rostock (DE); Hein, André, 18055 Rostock (DE)
(74) Vertreter: Galander, Marcus

(57) **Zusammenfassung**

Einführkatheter zum Setzen und Zurückziehen eines kardiovaskulären Implantats, insbesondere eines Stents oder einer Herzklappenprothese, mit einem langgestreckten Katheterkörper und einer am distalen Ende des Katheterkörpers angebrachten und distal reversibel aufweitbaren Katheterhülle, dadurch gekennzeichnet, dass die Katheterhülle eine Mehrzahl erster Magnetelemente aufweist, die über den Umfang verteilt sind, und im distalen Endbereich des Katheterkörpers mindestens ein zweites Magnetelement angeordnet ist, wobei die ersten Magnetelemente und das mindestens eine zweite Magnetelement derart ausgebildet und angeordnet sind, dass in Folge einer zwischen ihnen bestehenden Anziehungskraft die Katheterhülle aus einem aufgeweiteten Zustand wieder weitgehend in den Ursprungszustand zurückverformt wird.

## Beschreibung

Die Erfindung betrifft einen Einführkatheter zum Implantieren eines kardiovaskulären Implantats, insbesondere eines Stents oder einer Herzklappenprothese, mit einem inneren ersten Katheterschaft, welcher innerhalb von mindestens einem zweiten äußeren Katheterschaft angeordnet ist, wobei das Implantat im distalen Bereich des Einführkatheters auf dem inneren ersten Katheterschaft angeordnet ist, und wobei das distale Ende des zweiten äußeren Katheterschafts als distale reversibel aufweitbare Katheterhülle ausgeführt ist. Sie betrifft des Weiteren eine Katheterhülle eines solchen Einführkatheters und eine mit diesem gebildete Katheteranordnung.

Minimal invasive chirurgische Eingriffe gewinnen seit Jahren ständig an Bedeutung und sind beispielsweise zur Behandlung von Stenosen unverzichtbar. In letzter Zeit werden sie auch verstärkt bei der Implantation künstlicher Herzklappen eingesetzt. Geeignete Einführkatheter sind in großer konstruktiver Vielfalt bekannt und Gegenstand ständiger Weiterentwicklung. In den letzten Jahren wurde hier bei der Weiterentwicklung zu Kathetern besonderes Augenmerk geschenkt, die nicht nur das Setzen, sondern auch das Zurückziehen bzw. eine Repositionierung von kardiovaskulären Implantaten ermöglichen.

Für das Freisetzen (,Delivery') von Herzklappenstents werden heutzutage vorwiegend Katheter aus Kunststoffen bzw. Komposite eingesetzt deren Biegsamkeit und Flexibilität begrenzt sind. Gerade beim Implantieren (Freilassen und Zurückziehen, "Resheathen") von Herzklappenstents verursachen die Reaktionskräfte des Stents beim Zurückziehen in den Katheter starke Reaktionskräfte abhängig von der Steifigkeit des Implantats. Diese Reaktionskräfte können zu bleibenden Dehnungen der Katheterhülle führen, welche beim Entfernen des Katheters aus den Gefäßen unerwünscht sind.

Derartige Einführkatheter bestehen im Wesentlichen aus einem ersten, inneren Katheterschaft, auf dessen distalem Ende das Implantat angeordnet ist. Das Implantat und der erste, innere Katheterschaft sind von einem zweiten, äußeren Katheterschaft umgeben. Der distale Bereich des zweiten äußeren Katheterschafts, der das Implantat umgibt, wird häufig auch als Kapsel oder, wie im Folgenden, als Katheterhülle bezeichnet. Die Katheterhülle kann dabei aus dem gleichen Material wie der zweite äußere Katheterschaft oder aus einem anderen Material bestehen, welches mit dem zweiten äußeren Katheterschaft verbunden wird.

Hier wird unter der Positionsbezeichnung "proximal" ein näher zum Behandler liegender Teil des Einführkatheters bezeichnet. "Distal" bezeichnet entsprechend einen Teil des Einführkatheters, der sich weiter entfernt vom Behandler befindet.

Häufig sind die Implantate aus einem Formgedächtnismaterial ausgeführt. In diesen Fällen werden die Implantate während des Einführens an den Implantationsort durch die, sie umgebende, Katheterhülle in ihrer komprimierten Form gehalten. Durch Verschieben der Katheterhülle, beispielsweise nach proximal, verschwindet die Rückhaltekraft, die die Katheterhülle auf das Implantat ausübt, und das Implantat expandiert.

Vor diesem Hintergrund wurden spezielle Gestaltungen der Katheterhülle vorgeschlagen, die am distalen Ende des Katheter-Grundkörpers sitzt.

So offenbart die US 8,512,401 B2 eine Katheterhülle, die nach einer Aufweitung selbsttätig im Wesentlichen in ihren Ausgangszustand (nicht expandiert) zurückkehren soll. Hierbei sind in eine polymere Hülle langgestreckte und im nicht-expandierten Zustand in Längsrichtung des Katheters sich erstreckende Teile aus einer Gedächtnislegierung eingebettet, die nach einer Aufspreizung vermittels ihrer Memory-Eigenschaften die Rückkehr der Hülle in den Ausgangszustand bewirken. Zur Bildung der polymeren Hülle werden bestimmte Faltungen eines speziell vorgeformten Polymerfilms zur Umschließung der Metallteile vorgeschlagen.

Auch in der US 2011/0098804 A1 wird der Einsatz einer Gedächtnislegierung zur Konstruktion eines reversibel aufweitbaren distalen Katheterendes vorgeschlagen. In einer Ausführung hat das Katheterende einen stent-ähnlichen Aufbau und ist trichterförmig oder auch ballonartig reversibel verformbar.

Die WO 2013/155474 A1 offenbart mehrere vielgliedrige und konstruktiv aufwendige Ausführungen des distalen Katheterendes, bei denen ein Zurückführen des beim Freisetzen oder Resheathen eines Implantats aufgespreizten, stent-artig elastisch ausgeführten Katheterendes mittels einer Zugfadenanordnung aus mehreren Zugfäden erfolgt, die mit Streben der stent-artigen Konstruktion am Katheterende verbunden sind und sich längs durch den Katheter bis zu einem Betätigungselement am proximalen Ende erstrecken.

Die erwähnten Konstruktionen sind aufwendig und kostenintensiv und werfen teilweise auch Probleme hinsichtlich der Unterbringung in einem Einführkatheter der üblichen Dimensionen und/oder einer zuverlässigen Funktion unter klinischen Einsatzbedingungen auf.

Der Erfindung liegt daher die Aufgabe zugrunde, einen verbesserten Einführkatheter anzugeben, der insbesondere konstruktiv einfach aufgebaut und daher relativ kostengünstig zu fertigen ist und unter den relevanten Einsatzbedingungen zuverlässig funktioniert.

Diese Aufgabe wird durch einen Einführkatheter mit den Merkmalen des Anspruchs 1 gelöst. Zweckmäßige Ausgestaltungen der Erfindung sind Gegenstand der abhängigen Ansprüche.

Die Erfindung geht von der Überlegung aus, bei der Konstruktion des distalen Katheterendes sowohl auf komplizierte metallische Gerüstkonstruktionen als auch auf ähnlich komplexe Zugfadenanordnung zu verzichten und stattdessen ein grundsätzlich andersartiges Wirkprinzip für das Zurückführen des Katheterendes in seinen nicht-expandierten Ausgangszustand anzuwenden, welches auf einer geeigneten Ausgestaltung der Katheterhülle beruht. Weiterhin schließt sie die Überlegung ein, ein nicht-mechanisches Wirkprinzip anzuwenden. Dies führt schließlich zu dem Gedanken, dass die Katheterhülle eine Mehrzahl erster Magnetelemente aufweist, die über den Umfang verteilt sind, und im distalen Endbereich des inneren Katheterschafts mindestens ein zweites Magnetelement angeordnet ist. Erfindungsgemäß sind die ersten Magnetelemente und das mindestens eine zweite Magnetelement derart ausgebildet und angeordnet, dass in Folge einer zwischen ihnen bestehenden Anziehungskraft die Katheterhülle aus einem aufgeweiteten Zustand wieder weitgehend in den Ursprungszustand zurückverformt wird. Alternativ wird das distale Ende des Außenschafts derart ausgestaltet bzw. ausgeformt, dass die Form des distalen Endes ein Schieben des Außenschafts über ein teilweise freigesetztes Implantat erleichtert.

Mit der Erfindung wird ein grundsätzlich einfach aufgebauter und daher kostengünstig herstellbarer Einführkatheter bereitgestellt, dessen einfache Konstruktion wenig störanfällig und dessen Funktion daher auch unter klinischen Bedingungen als zuverlässig anzusehen ist. Zudem ist die Handhabung dieses Einführkatheters jedenfalls in einfacheren Realisierungsformen sehr leicht.

In einer Ausführung der Erfindung ist das oder jedes zweite Magnetelement ein Permanentmagnet. In einer alternativen Ausführung weist das zweite Magnetelement eine über eine elektrische Zuleitung ansteuerbare Magnetspule auf.

In einer weiteren Ausführung ist das oder jedes zweite Magnetelement in einem Lumen des Katheterkörpers in Längsrichtung verschieblich angeordnet, und am proximalen Ende des Katheterkörpers ist ein Betätigungselement zum Verschieben des oder jedes zweiten Magnetelements vorgesehen. Das verschiebliche zweite Magnetelement kann etwa auf einem Schub- und Zugdraht oder einem funktionsgleichen dünnen Kunststoff- oder Karbonstab o.ä. angeordnet sein, dessen Ende durch ein separates Bedienelement in einem Katheter-Griffstück gebildet ist.

In einer weiteren Ausführung der Erfindung sind die ersten Magnetelemente als in die Katheterhülle fein verteilt eingebettete magnetische Partikel ausgebildet. In alternativen Ausführungen können die ersten Magnetelemente auch in eine zweilagige Folien-Ausführung der Katheterhülle eingeschweißte oder auf eine einfache Polymer-Lage aufgeklebte kleine Magnetkörper sein.

In weiteren Ausführungen der Erfindung weist die Katheterhülle an ihrem distalen Ende eine Mehrzahl von, insbesondere über den Umfang gleichmäßig verteilten, Finger- oder Spitzenabschnitten auf. Speziell kann hierbei die Länge der Finger- oder Spitzenabschnitte größer als deren Breite, insbesondere größer als deren zweifache Breite, an der Basis derselben sein. Hierbei ist in einer Ausgestaltung in jedem Finger- oder Spitzenabschnitt mindestens ein erstes Magnetelement angeordnet.

Gemäß einem relativ selbstständigen zweiten Aspekt der Erfindung wird ein Einführkatheter zum Setzen und Zurückziehen eines kardiovaskulären Implantats, eines Stents oder einer Herzklappenprothese, vorgeschlagen, der sich dadurch auszeichnet, dass die Katheterhülle eine geschlossene Umfangsfläche hat, deren distale Begrenzung eine trapez- oder wellenförmige Kontur oder sichelförmige Einbuchtungen aufweist, wobei die Tiefe der Wellentäler oder Trapeze bzw. Einbuchtungen insbesondere kleiner als die Hälfte ihrer Breite ist.

Gemäß einem zweiten Aspekt der Erfindung wird im Wesentlichen die geometrische Form des Umfangs der Katheterhülle derart verändert, dass ein Zurückschieben der Katheterhülle nach distal über ein teilweise freigesetztes Implantat erleichtert wird. Die vorangehend beschriebenen Ausbuchtungen ermöglichen eine teilweise Ausdehnung des Implantats an der Seite der Einbuchtung. Vereinfacht ausgedrückt ist die Katheterhülle so ausgestaltet, dass sie an den Stellen ohne Einbuchtung einen Bereich des Implantats überdeckt, während an den Stellen der Ausbuchtung das Implantat noch in seinem ausgedehnten Zustand ist. Entsprechend muss beim Zurückschieben der Katheterhülle nach distal das teilweise freigesetzte Implantat nicht über seinen gesamten Umfang gleichzeitig komprimiert werden. Dies reduziert die zum Zurückschieben notwendige Kraft und erleichtert so dass erneute Komprimieren des teilweise freigesetzten Implantats.

Vorteile und Zweckmäßigkeiten der Erfindung ergeben sich im Übrigen aus der nachfolgenden skizzenartigen Beschreibung von Ausführungsbeispielen anhand der Figuren. Von diesen zeigen:
- Fig. 1: eine perspektivische Darstellung des Endes eines erfindungsgemäßen Einführkatheters mit daraus weitgehend freigesetztem Implantat,
- Fig. 2A bis 2D: skizzenartige Darstellungen von Katheterhüllen gemäß Ausführungsbeispielen der Erfindung und
- Fig. 3A bis 3C: weitere skizzenartige Darstellungen von Katheterhüllen gemäß Ausführungsbeispielen der Erfindung.

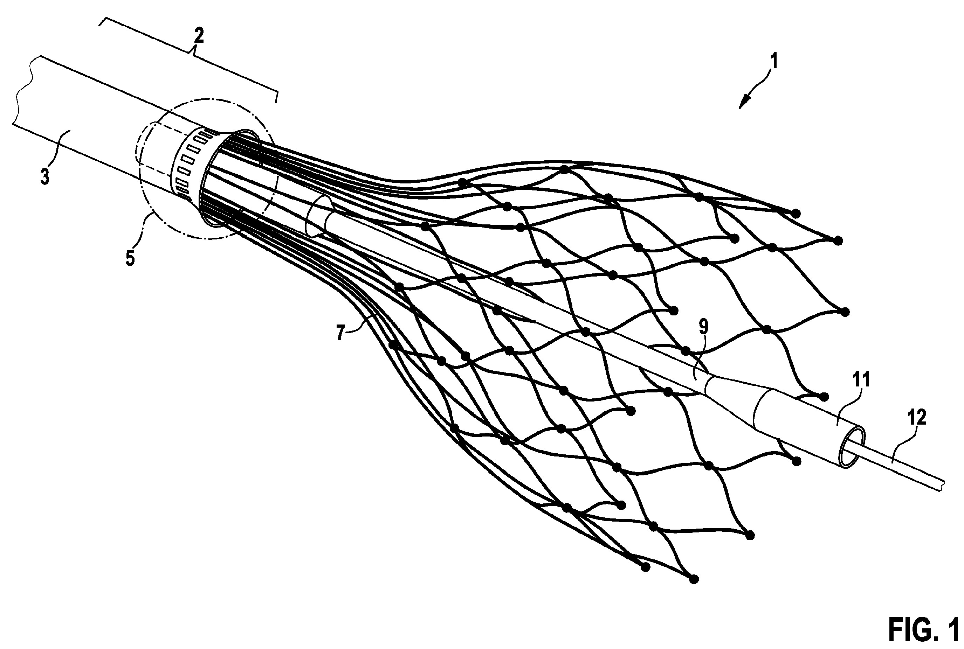

Fig. 1 ist eine Ausschnittdarstellung einer Einführkatheteranordnung 1, die einen Einführkatheter 2 mit einem äußeren Katheterschaft 3 und mit der Katheterhülle 5 am distalen Ende sowie ein aus dem Einführkatheter heraus teilweise expandiertes kardiovaskuläres Implantat 7 darstellt. Ein derartiges Implantat 7 kann ein selbstexpandierender Stent oder ein Herzklappenimplantat mit einem selbstexpandierenden Grundkörper sein. Zu erkennen ist auch ein aus dem distalen Katheterende und der Katheterhülle 5 distal herausragender Führungsdraht 12, sowie der erste innere Katheterschaft 9, der das Implantat trägt, und einem an dessen distalem Ende sitzenden Permanentmagneten 11. Während der Katheterkörper 3 und das Implantat 7 als solche in vielerlei Ausführungen aus dem Stand der Technik bekannt sind, weist die Katheterhülle 5 eine weiter unten beschriebene spezielle Konstruktion unter Einschluss von Magnetkörpern auf. Der im ersten inneren Schaft befestigte oder eingelassene Permanentmagnet 11 dient dazu, im Zusammenwirken mit den Magneten an/in der Katheterhülle eine Rückkehr derselben aus dem expandierten Zustand im Wesentlichen in den Ausgangszustand zu bewirken.

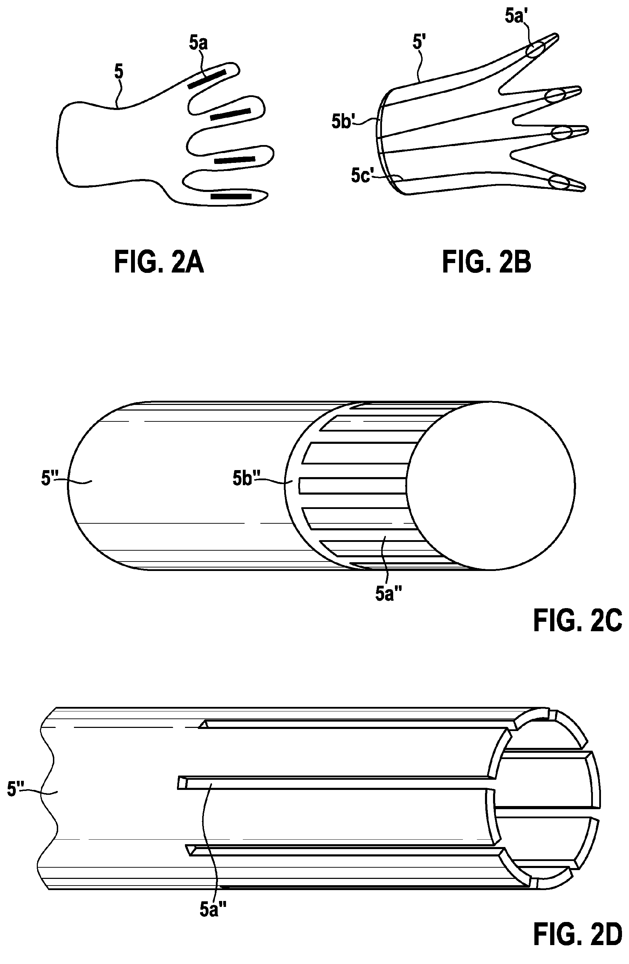

Fig. 2A und 2B sind schematische Seitenansichten von Ausführungsformen der Katheterhülle 5, 5' mit daran/darin angebrachten Permanentmagneten 5a bzw. 5a'. Während die Katheterhülle 5 aus Fig. 2A am distalen Ende im Wesentlichen fingerartig geschlitzt ist und die Magnetelemente 5a als längliche dünne Stäbe bzw. Plättchen ausgeführt sind, ist die distale Begrenzungslinie der Katheterhülle 5' nach Fig. 2B zickzackförmig, weist also über den Umfang verteilte Spitzen auf, und die Magnete 5a' haben hier eine elliptische bzw. ovale Grundform. Zudem sind in Fig. 2B ein proximaler Endring 5b' und in Längsrichtung in die Spitzen hinein verlaufende Verstärkungsstreben 5c' als zusätzliche mechanische Konstruktionselemente gezeigt. Es versteht sich, dass auch die Katheterhülle 5 nach Fig. 2A mit solchen Konstruktionselementen aufgebaut sein kann.

Fig. 2C und 2D sind skizzenartige perspektivische Ansichten einer Katheterhülle 5", die - anders als die Ausführungen nach Fig. 2A und 2B - ein unstrukturiertes, ringartig geschlossenes distales Ende hat und in die mit gleichen Abständen voneinander schmale längliche Magnetelemente 5a" eingebettet sind. Die Magnetelemente sind jeweils parallel zur Längsachse der Katheterhülle ausgerichtet. Bei der Konfiguration nach Fig. 2c sind sie an ihrem proximalen Ende mittels eines umlaufenden Magnetringes 5b" miteinander verbunden. Da die Katheterhülle nach Fig. 2C und 2D bis zum distalen Ende rundum geschlossen ist, ist die Ausführung mit einem besonders hochgradig elastischen Kunststoffmaterial aus derzeitiger Sicht bevorzugt.

Im Übrigen ist die Katheterhülle aus für diesen Zweck bekannten Materialien, etwa PEBAX oder einer anderen geeigneten Kunststofffolie, gefertigt. Sie kann einlagig mit aufgesetzten Magneten oder zweilagig mit zwischen beide Lagen eingebetteten Magneten konstruiert sein. Die Permanentmagnete sind umgekehrt gepolt wie der zentral im ersten inneren Katheterschaft angeordnete Permanentmagnet 11 (Fig. 1), und sie können aus bekannten bio-verträglichen Materialien bestehen, wie etwa einem magnetischen Kunststoff auf TCNE-Basis oder Werkstoffen ähnlich den aus der Dentalprothetik bekannten Titanmagnetics-Systemen.

Alternativ zum Vorsehen von einzelnen kleinen Magnetkörpern an der Katheterhülle ist auch der Einsatz eines insgesamt mit Magnetpartikeln gefüllten Hüllenmaterials möglich, welches zusätzlich mit einer Faserverstärkung oder weiteren Füllmitteln versetzt sein kann.

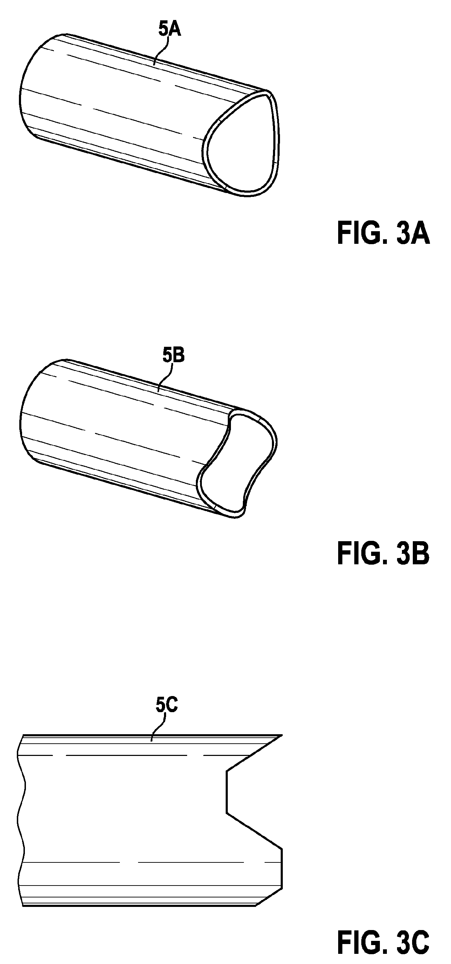

Fig. 3A und 3B zeigen in schematischen, perspektivischen Darstellungen Außenansichten weiterer Katheterhüllen 5A und 5B, und Fig. 3C zeigt eine schematische Seitenansicht einer dritten Katheterhülle 5C, welche einen zweiten relativ unabhängigen Aspekt der Erfindung realisieren. Während die Katheterhüllen 5A und 5B nach Fig. 3A und 3B eine wellen- bzw. bogen- oder sichelförmig geschwungene distale Begrenzungslinie haben, ist die distale Begrenzungslinie bei der Katheterhülle 5C nach Fig. 3C mit einem trapezförmigen Profil versehen. Diese und ähnliche (nicht gezeigte) Begrenzungslinien-Profile begünstigen ein leichtes Aufspreizen und Zurückverformen der Katheterhülle weitgehend in den Ausgangszustand. Sie können bei geeigneter Wahl eines elastischen Hüllenmaterials als selbstständige Lösungen, aber auch in Kombination mit der oben beschriebenen Lösung mit einander anziehenden Permanentmagneten realisiert sein.

Im Übrigen ist die Ausführung der Erfindung auch in einer Vielzahl von Abwandlungen der hier gezeigten Beispiele und weiter oben hervorgehobenen Aspekten der Erfindung möglich.

## Patentansprüche

1. Einführkatheter (2) zum Implantieren eines kardiovaskulären Implantats (7), insbesondere eines Stents oder einer Herzklappenprothese, mit einem inneren ersten Katheterschaft (9), welcher innerhalb von mindestens einem zweiten äußeren Katheterschaft (3) angeordnet ist, wobei das Implantat im distalen Bereich des Einführkatheters auf dem inneren ersten Katheterschaft angeordnet ist, und wobei das distale Ende des zweiten äußeren Katheterschafts als distale reversibel aufweitbare Katheterhülle (5; 5': 5A: 5B: 5C) ausgeführt ist,
**dadurch gekennzeichnet, dass**
die Katheterhülle eine Mehrzahl erster Magnetelemente (5a; 5a') aufweist, die über den Umfang verteilt sind, und im distalen Endbereich des inneren ersten Katheterschafts mindestens ein zweites Magnetelement (11) angeordnet ist,
wobei die ersten Magnetelemente und das mindestens eine zweite Magnetelement derart ausgebildet und angeordnet sind, dass in Folge einer zwischen ihnen bestehenden Anziehungskraft die Katheterhülle aus einem aufgeweiteten Zustand wieder weitgehend in den Ursprungszustand zurückverformt wird.

2. Einführkatheter nach Anspruch 1, wobei das oder jedes zweite Magnetelement (11) ein Permanentmagnet ist.

3. Einführkatheter nach Anspruch 1, wobei das zweite Magnetelement eine über eine elektrische Zuleitung ansteuerbare Magnetspule aufweist.

4. Einführkatheter nach einem der vorangehenden Ansprüche, wobei das oder jedes zweite Magnetelement (11) in einem Lumen des Katheterkörpers in Längsrichtung verschieblich angeordnet ist und am proximalen Ende des Katheterkörpers ein Betätigungselement zum Verschieben des oder jedes zweiten Magnetelements vorgesehen ist.

5. Einführkatheter nach einem der vorangehenden Ansprüche, wobei die ersten Magnetelemente als in die Katheterhülle fein verteilt eingebettete magnetische Partikel ausgebildet sind.

6. Einführkatheter nach einem der vorangehenden Ansprüche, wobei die Katheterhülle (5; 5') an ihrem distalen Ende eine Mehrzahl von, insbesondere über den Umfang gleichmäßig verteilten, Finger- oder Spitzenabschnitten aufweist.

7. Einführkatheter nach Anspruch 6, wobei die Länge der Finger- oder Spitzenabschnitte größer als deren Breite, insbesondere größer als deren zweifache Breite, an der Basis derselben ist.

8. Einführkatheter nach Anspruch 6 oder 7, wobei in jedem Finger- oder Spitzenabschnitt mindestens ein erstes Magnetelement (5a; 5a') angeordnet ist.

9. Einführkatheter (2) zum Implantieren eines kardiovaskulären Implantats (7), insbesondere eines Stents oder einer Herzklappenprothese, mit einem inneren ersten Katheterschaft (9), welcher innerhalb von mindestens einem zweiten äußeren Katheterschaft (3) angeordnet ist, wobei das Implantat im distalen Bereich des Einführkatheters auf dem inneren ersten Katheterschaft angeordnet ist, und wobei
das distale Ende des zweiten äußeren Katheterschafts als distale reversibel aufweitbare Katheterhülle (5; 5': 5A: 5B: 5C) ausgeführt ist,
insbesondere nach einem der Ansprüche 1 bis 5,
**dadurch gekennzeichnet, dass**
die Katheterhülle eine geschlossene Umfangsfläche hat, deren distale Begrenzungslinie eine trapez- oder wellenförmige Kontur oder sichelförmige Einbuchtungen aufweist, wobei die Tiefe der Wellentäler oder Trapeze bzw. Einbuchtungen kleiner als die Hälfte ihrer Breite ist.

10. Katheterhülle (5; 5'; 5A; 5B; 5C) zur Bildung eines Einführkatheters (2) nach einem der vorangehenden Ansprüche.

11. Einführkatheteranordnung (1) mit einem Einführkatheters (2) nach einem der Ansprüche 1 bis 9 und einem an die distal auf diesem angeordnete Katheterhülle (5; 5'; 5A; 5B; 5C) angepassten kardiovaskulären Implantat (7), insbesondere einem Stent oder einer Herzklappe.
